# EUROPEAN PATENT SPECIFICATION

(11) **EP 2 047 691 B1**
(45) Date of publication and mention of the grant of the patent: **16.01.2013**
(21) Application number: 07736308.3
(22) Date of filing: 10.05.2007
(51) Int. Cl.: A61M 29/00

(54) **SYSTEMS FOR TREATING A VESSEL USING FOCUSED FORCE**
SYSTEME ZUM BEHANDELN EINES GEFÄSSES DURCH VERWENDUNG EINER FOKUSSIERTEN KRAFT
SYSTÈMES POUR TRAITER UN VAISSEAU AU MOYEN D'UNE FORCE CONCENTRÉE

(30) Priority: 11.05.2006 US 431918; 03.10.2006 WO PCT/IL2006/001150
(43) Date of publication of application: 15.04.2009
(73) Proprietor: Y Med Inc., Tempe, AZ 85281 (US)
(72) Inventor: SOLAR, Ronald J., San Diego, CA 92131 (US); SHAKED, Yoav, 42823 Tzoran (IL); LIEBER, Glen, Poway, California 92064 (US)
(74) Representative: HOFFMANN EITLE
(86) International application number: PCT/IL2007/000568
(87) International publication number: WO 2007/132447

(56) References cited:
- WO-A-95/26777
- US-A- 5 520 647
- US-A1- 2003 125 761
- US-A1- 2005 154 440
- US-B2- 6 579 312
- US-B2- 6 579 312

## Description

### FIELD OF THE INVENTION

The present invention relates to a device for treating a vessel using focused force, to aid in cracking of difficult lesions.

### BACKGROUND OF THE INVENTION

Balloon dilatation catheters are used to treat lesions in vessels. However, difficulties are encountered in navigating tortuous anatomy and safely crossing very tight lesions. Moreover, some lesions are difficult to crack using just a balloon, and require a focused force to enable cracking of the lesion at safe inflation pressures.

An example of a system used to provide enhanced force is disclosed in U.S. Patent Number 6,394,995 to Solar et al. Disclosed therein is a system having a flexible advancement member with a tracking member slidable over a guidewire, and a balloon having a distal end attached to the tracking member. However, this type of system provides limited focused force, does not address bifurcation lesions, and lacks pushability and maneuverability. Further examples of guidewire systems are disclosed in US5520647. US2003/125761 and US6579312. WO-A-95/26777 discloses a catheter for performing multiple procedures including stent placement and the like. US 2005/154440 A1 discloses a balloon catheter having an advancement wire with a textured surface extending along an outer surface of an inflatable section of the catheter balloon.

It is therefore an object of the present invention to provide enhanced balloon dilatation catheter systems with improved maneuverability and multiple treatment options.

### SUMMARY OF THE INVENTION

The invention is defined in the appended claims. According to one aspect of the present invention, mere is provided a device for introduction into a vessel. The device includes a main elongated element having a main elongated element proximal end and a main elongated element distal end, a balloon positioned at the main elongated element distal end, an auxiliary elongated element having an auxiliary elongated element proximal end and an auxiliary elongated element distal end, the auxiliary elongated element distal end positioned proximal to the balloon, and a core wire including an internal core wire portion positioned within the main elongated element and attached to the main elongated element at a core wire attachment point and an external core wire portion positioned distally with respect to the internal core wire portion, the external core wire portion external to and running alongside the balloon.

According to features of the present invention, in some embodiments a distal connecting element is positioned at a distal end of the balloon and may be rotationally spaced from or aligned with the auxiliary elongated element. In other embodiments, a fixed wire is positioned at the distal end of the balloon. The device may be over-the-wire or rapid exchange, as these terms are known in the art, or a combination thereof. In some embodiments, the external core wire has a coil for preventing slippage of the balloon with respect to the lesion. In some embodiments, an occlusion balloon is positioned proximal to the auxiliary elongated element distal end.

In accordance with additional aspects of the present invention, there is provided a device for introduction into a vessel. The device includes a main elongated element having a main elongated element proximal end and a main elongated element distal end, a balloon positioned at the main elongated element distal end, an auxiliary elongated element having a proximal and a distal end, the auxiliary elongated element distal end positioned proximal to the balloon, and a distal connecting element positioned at a distal end of the balloon, wherein the distal connecting element is at a rotational distance from the auxiliary elongated element.

In accordance with additional examples, there is provided a method for treating a vessel. The method includes providing a device having a main elongated element, a balloon at a distal end of the device, an auxiliary elongated element wherein a distal end of the auxiliary elongated element is proximal to the balloon, and a wire attached to the device and positioned alongside the balloon and on an opposite side of the balloon as the auxiliary elongated element and a distal connecting element at a distal end of the balloon, inserting a tracking guidewire into the vessel, backloading the tracking guidewire into the distal connecting element, advancing the device over the tracking guidewire until the distal end of the device is adjacent to the lesion, advancing a second guidewire through the auxiliary elongated element, and inflating the balloon so as to push at least the attached wire and the tracking guidewire against different sides of a lesion in the vessel.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The above and further advantages of the present invention may be better understood by referring to the following description in conjunction with the accompanying drawings in which:
FIG. 1A is a schematic illustration of a system for treatment of a vessel, in accordance with embodiments of the present invention;
FIGS. 1B-1D are cross-sectional illustrations of the system of FIG. 1A;
FIG. 1E is a perspective illustration of the system of FIG. 1A;
FIG. 1F is a schematic illustration of the system of FIG. 1A, with an occlusion balloon;
FIG. 1G is a cross-sectional illustration of the system of FIG. 1F;
FIG. 2 is a schematic illustration of a system for treatment of a vessel, in accordance with other embodiments of the present invention;
FIG. 3 is a schematic illustration of a system for treatment of a vessel, in accordance with yet additional embodiments of the present invention;
FIG. 4 is a schematic illustration of a system for treatment of a vessel, in accordance with yet additional embodiments of the present invention;
FIGS. 5A and 5B are illustrations of a core wire, in accordance with embodiments of the present invention;
FIGS. 6A-6D are cross-sectional illustrations of a distal portion of the systems of FIGS. 1-4;
FIGS. 7A-7F are schematic illustrations of the steps of a method of treating a vessel, in accordance with embodiments of the present invention;
FIGS. 8A-8C are schematic illustrations of the steps of a method of treating a vessel, in accordance with additional embodiments of the present invention;
FIGS. 9A-9C are schematic illustrations of the steps of a method of treating a vessel, in accordance with additional embodiments of the present invention; and
FIGS. 10A-10C are schematic illustrations of the steps of a method of treating a bifurcated vessel, in accordance with additional embodiments of the present invention.

It will be appreciated that for simplicity and clarity of illustration, elements shown in the drawings have not necessarily been drawn accurately or to scale. For example, the dimensions of some of the elements may be exaggerated relative to other elements for clarity or several physical components may be included in one functional block or element. Further, where considered appropriate, reference numerals may be repeated among the drawings to indicate corresponding or analogous elements. Moreover, some of the blocks depicted in the drawings may be combined into a single function.

### DETAILED DESCRIPTION

In the following detailed description, numerous specific details are set forth in order to provide a thorough understanding of the present invention. It will be understood by those of ordinary skill in the art that the present invention may be practiced without these specific details. In other instances, well-known methods, procedures, components and structures may not have been described in detail so as not to obscure the present invention.

The present invention is directed to a device for treatment of a vessel using focused force. The principles and operation there of according to the present invention may be better understood with reference to the drawings and accompanying descriptions.

Before explaining at least one embodiment of the present invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein are for the purpose of description and should not be regarded as limiting.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

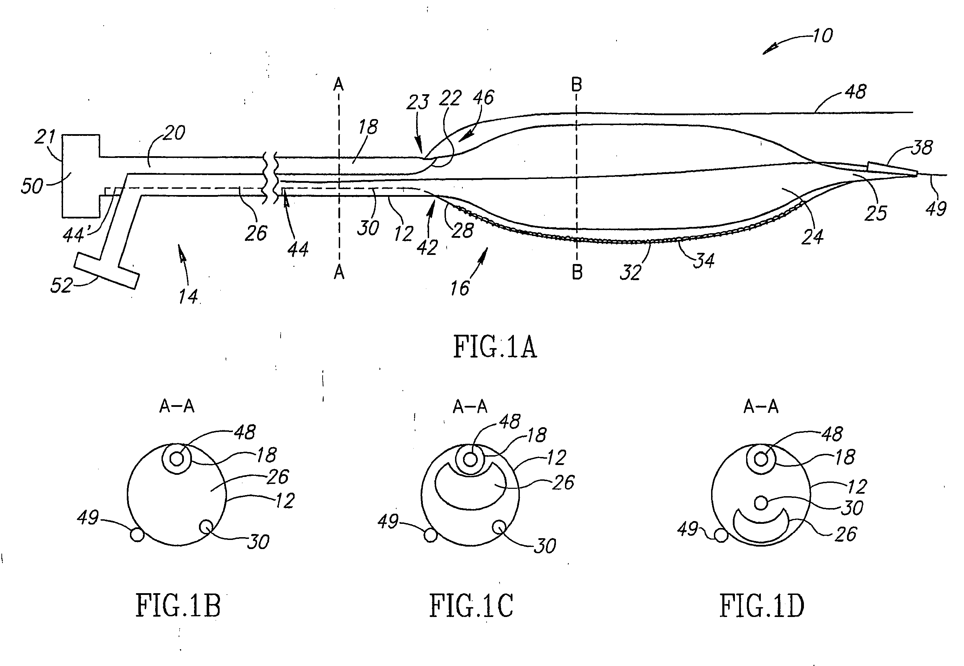

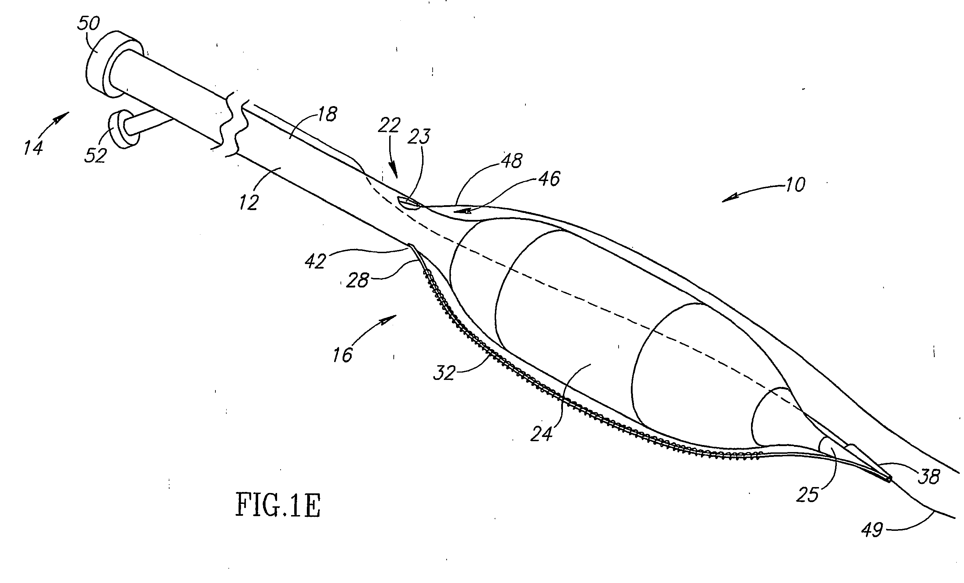

Reference is now made to FIGS. 1A and 1E, which are a schematic and perspective illustration, respectively, of a system 10 for treatment of a vessel, in accordance with embodiments of the present invention. System 10 includes a main elongated element 12 having a proximal end 14 and a distal end 16. In some embodiments of the present invention, main elongated element 12 is a catheter shaft. A balloon 24 is positioned at distal end 16 of main elongated element 12. Balloon 24 can be comprised of a variety of diameters, ranging from 1.25-10.0 mm, for example, and a variety of lengths, ranging from 10 mm to 30 cm, for example. Long balloons may be particularly useful for treating peripheral lesions, which often have long diseased portions. System 10 further includes an auxiliary elongated element 18 configured to receive a guidewire 48 therethrough. Auxiliary elongated element 18 has a proximal end 20 with a proximal exit point 21 for guidewire 48 and a distal end 22 with a distal exit point 23 for guidewire 48. In some embodiments, at least a portion of auxiliary elongated element 18 is positioned within main elongated element 12 so as to reduce the outer profile of system 10. Distal end 22 of auxiliary elongated element 18 is proximal to balloon 24 such that guidewire 48, when positioned through auxiliary elongated element 18, exits distal exit point 23 and runs alongside and external to balloon 24. This configuration provides for a focused force element alongside balloon 24, as will be described further hereinbelow. In some embodiments, such as the one shown in FIGS. 1A and 1E, auxiliary elongated element 18 runs along the length of main elongated element 12 to a proximal guidewire port 50. This configuration provides an over-the-wire type of configuration. In one embodiment, guidewire 48 positioned through distal exit point 23 forms a crotch point 46 at or near a proximal end of balloon 24. The presence of a crotch point may be useful, for example, for anchoring system 10 within a side branch to avoid slippage within the vessel to be treated, or to provide for precise positioning of system 10 at a bifurcation.

In some embodiments, main elongated element 12 is stiffer proximally than distally. This may be accomplished, for example, by using a metal hypotube in the proximal portion and a polymer or other flexible material in the distal portion. This configuration provides more flexibility at the distal end to allow for easier maneuverability through tortuous vessels, while maintaining rigidity at a proximal end for pushability. However, if the distal portion of main elongated element 12 is too flexible, it will be difficult to push through the vessels. Thus, system 10 further includes a core wire 28, which provides enhanced pushability of system 10 without significantly reducing the flexibility of system 10. Core wire 28 is provided in the flexible portion, and may terminate at the stiff portion when no longer needed for rigidity. In other embodiments, main elongated element 12 is relatively flexible along all or most of its length, by using a flexible polymer or other flexible material to form main elongated element 12. In these embodiments, core wire 28 may run along an entire length of main elongated element 12 and may vary in diameter along the length so as to provide increased rigidity at proximal end 14. In some embodiments, the flexible shaft may also be braided or otherwise strengthened to provide sufficient rigidity.

In embodiments of the present invention, core wire 28 has a portion positioned within main elongated element 12, referred to herein as internal core wire portion 30, and a portion positioned external to main elongated element 12, referred to herein as external core wire portion 32. Internal core wire portion 30 is proximal to external core wire portion 32, and is attached to main elongated element 12 at an internal core wire attachment point 44. For embodiments wherein main elongated element 12 is comprised of a relatively flexible distal portion and a relatively rigid proximal portion, internal core wire attachment point 44 is located at an interface between the stiff proximal portion and the flexible distal portion, for example, a distal end of the hypotube. In embodiments wherein main elongated element is mostly or completely comprised of flexible material, internal core wire attachment point 44 is located at proximal end 14 of system 10. However, it should be readily apparent that internal core wire attachment point 44 may be located at any location along the length of main elongated element 12. Moreover, multiple internal core wire attachment points 44 are included. At a location proximal to balloon 24, internal core wire portion 30 exits main elongated element 12 and becomes external core wire portion 32. This location is referred to herein as a core wire exit point 42. In one embodiment, core wire exit point 42 is at a distal end of main elongated element 12. In other embodiments, core wire exit point 42 is at other locations along main elongated element 12 (but in most cases proximal to balloon 24). Distal to core wire exit point 42, external core wire portion 32 is positioned alongside balloon 24, and a distal end of external core wire portion 32 is attached to a distal tip 25 of balloon 24. Several attachment or bonding locations provide transmission of forces through the length of the catheter, and thus enhance overall torquability and rotatability. In particular, bonding can be done at any or all of the following locations: at distal tip 25 of balloon 24, at core wire exit point 42, and at internal core wire attachment point 44. Additional attachment points may be included as well. It should be noted that the use of an internal core wire makes it possible to have a longer flexible (polymeric or other) portion or even a completely flexible shaft, enhancing overall flexibility of system 10.

System 10 further includes a distal connecting element 38 at distal tip 25 of balloon 24. Distal connecting element 38 is a short rail, ranging in length from 2-20 mm, and may be bonded to distal tip 25 such that the proximal end of distal connecting element 38 is distal to balloon 24. A three-way bond may be used to attach distal connecting element 38, balloon 24 and external core wire portion 32, all together. Distal connecting element 38 may be tapered toward its distal end to facilitate passage through tight stenoses. Distal connecting element 38 is positioned at a rotational distance from auxiliary elongated element 18 and from external core wire portion 32, and is configured to hold a tracking guidewire 49 therethrough. In some embodiments, distal connecting element 38, auxiliary elongated element 18 and external core wire portion 32 are positioned approximately 120° from one another. In other embodiments, other rotational distances may be used, such that there is some rotational separation between them. In this way, guidewire 48, tracking guidewire 49 and core wire 32 may all lie alongside balloon 24 at different rotational positions along balloon 24 when balloon 24 is in its expanded state. Although the separations between guidewire 48, tracking guidewire 49 and core wire 32 are not required to be any specific amounts, it should be apparent that the distances between them should be sufficient to provide separate wires alongside several different areas of balloon 24. Each of these wires can then provide a focused force to help crack difficult lesions, as will be explained further hereinbelow. It should be noted that in some embodiments, guidewire 48 and tracking guidewire 49 may be of different sizes.

Reference is now made to FIGS. 1B-1D, which are cross-sectional illustrations of system 10 shown at section A-A, in accordance with several embodiments of the present invention. As shown in FIG. 1B, an interior portion of main elongated element 12 serves as an inflation lumen 26, providing fluid communication between an inflation port 52 located at proximal end 14 of main elongated element 12 and balloon 24 located at distal end 16 of main elongated element 12. In some embodiments, a portion of the interior of main elongated element 12 is sectioned off for use as inflation lumen 26, as shown in FIG. 1C and in FIG 1D, wherein only the sectioned off inflation lumen 26 is in fluid communication with inflation port 52. Auxiliary elongated element 18 is positioned within main elongated element along an edge thereof. The cross-sectional views of FIGS. 1B-1D show auxiliary elongated element 18 with guidewire 48 positioned therein. Internal core wire portion 30 is positioned within main elongated element 12. In some embodiments, as shown in FIGS. 1B and 1C, internal core wire portion 30 is positioned along an edge of main elongated element 12. In other embodiments, as shown in FIG 1D, internal core wire portion 30 is positioned in a center of main elongated element 12. It should be readily apparent, however, that at core wire attachment point 44 and at core wire exit point 42, the core wire is in contact with or close proximity to an edge of main elongated element 12. Tracking guidewire 49 is shown external to main elongated element 12.

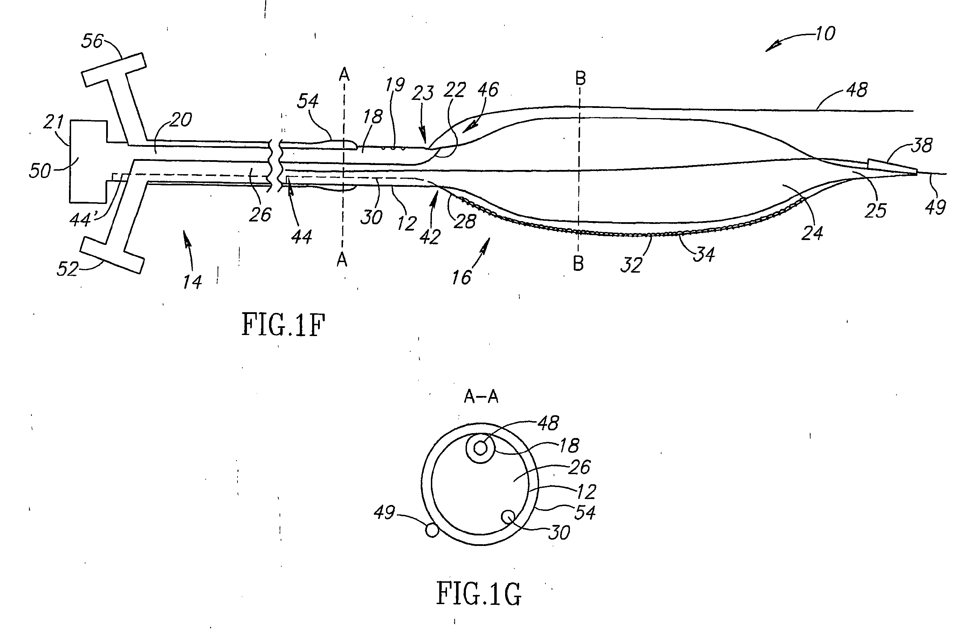

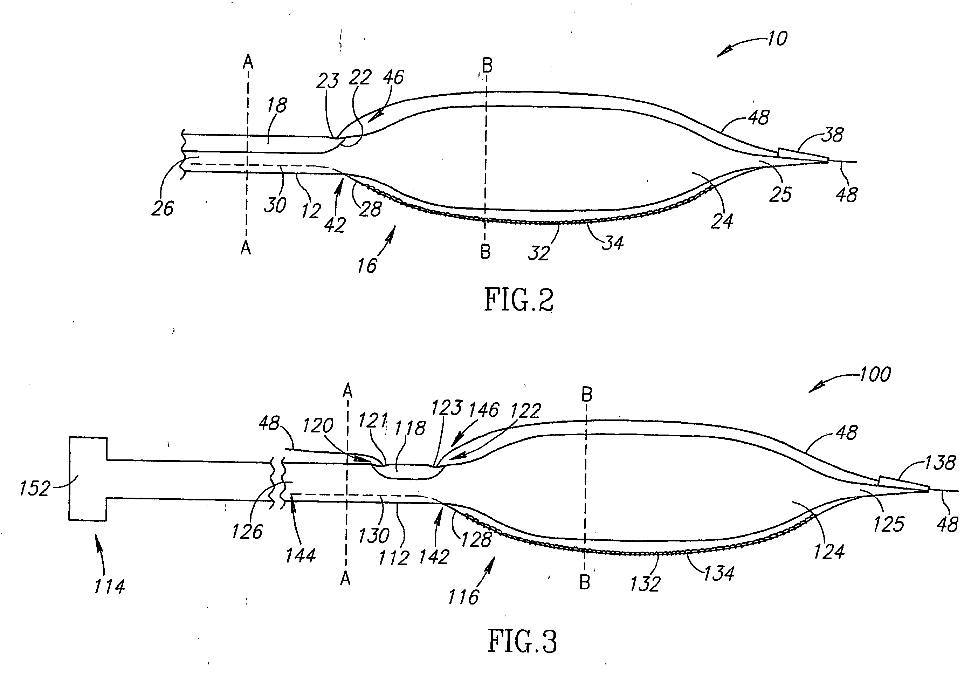

Reference is now made to FIGS. 1F and 1G, which are schematic and cross-sectional illustrations of system 10 further including an occlusion balloon 54. Occlusion balloon 54 is positioned around main elongated element 12 and is proximal to auxiliary element distal exit point 23. Occlusion balloon 54 may be used to temporarily occlude blood flow proximal to occlusion balloon 54, and to enable introduction of an item or a substance into the vessel at the lesion site via auxiliary elongated element 18. In some embodiments, the item is a treatment device, such as a guidewire with an ablation tip or any other treatment device. In some embodiments, the substance is contrast media. In other embodiments, the substance is a therapeutic drug or medicated solution. In some embodiments, multiple ports 19 may be included on auxiliary elongated element 18, distal to occlusion balloon 54. These multiple ports 19 may enable spraying of a substance such as contrast media, drugs, medicated solutions, etc. Reference is now made to FIG. 2, which is a schematic illustration of system 10, wherein distal connecting element 38 is aligned with auxiliary elongated element 18, such that guidewire 48 may be positioned through distal connecting element 38 and further through auxiliary elongated element 18, and out through auxiliary elongated element proximal exit point 21. Thus, only one guidewire is used in the configuration shown in FIG. 2. This design provides a single guidewire enclosure split into two sections - one at the distal end and one at the proximal end of balloon 24 - in order to reduce the profile of system 10 in the vicinity of balloon 24 during introduction of system 10 into a vessel. Guidewire 48, while positioned within distal connecting element 38 and auxiliary elongated element 18, can serve as a focused force to help crack difficult lesions and may also be used as a tracking guidewire for advancing system 10 into the vessel.

Reference is now made to FIG. 3, which is a schematic illustration of a system 100, in accordance with additional embodiments of the present invention. System 100 includes a main elongated element 112 having a proximal end 114 and a distal end 116. In some embodiments of the present invention, main elongated element 112 is a catheter shaft. A balloon 124 is positioned at distal end 116 of main elongated element 112. Balloon 124 can be comprised of a variety of diameters, ranging from 1.25-10.0 mm, for example, and a variety of lengths, ranging from 10 mm to 30 cm, for example. Long balloons may be particularly useful for treating peripheral lesions, which often have long diseased portions. System 100 further includes an auxiliary elongated element 118 configured to receive a guidewire 48 therethrough. Auxiliary elongated element 118 has a proximal end 120 with a proximal exit point 121 for guidewire 48 and a distal end 122 with a distal exit point 123 for guidewire 48. In some embodiments, at least a portion of auxiliary elongated element 118 is positioned within main elongated element 112 so as to reduce the outer profile of system 100. Distal end 122 of auxiliary elongated element 118 is proximal to balloon 124 such that guidewire 48, when positioned through auxiliary elongated element 118, exits distal exit point 123 and runs alongside and external to balloon 124. This configuration provides for a focused force element alongside balloon 124, as will be described further hereinbelow. In some embodiments, such as the one shown in FIG. 3, auxiliary elongated element 118 is relatively short, extending 5-30 cm, and in some embodiments approximately 20 cm. This configuration enables rapid exchange in cases when system 100 may need to be retracted and a different device reinserted over guidewire 48. In one embodiment, guidewire 48 positioned through distal exit point 123 forms a crotch point 146 at or near a proximal end of balloon 124. The presence of a crotch point may be useful, for example, for anchoring system 100 within a side branch to avoid slippage within the vessel to be treated, or to provide for precise positioning of system 100 at a bifurcation.

In some embodiments, main elongated element 112 is stiffer proximally than distally. This may be accomplished, for example, by using a metal hypotube in the proximal portion and a polymer or other flexible material in the distal portion. This configuration provides more flexibility at the distal end to allow for easier maneuverability through tortuous vessels, while maintaining rigidity at a proximal end for pushability. However, if the distal portion of main elongated element 112 is too flexible, it will be difficult to push through the vessels. Thus, system 100 further includes a core wire 128, which provides enhanced pushability of system 100 without significantly reducing the flexibility of system 100. Core wire 128 is provided in the flexible portion, and may terminate at the stiff portion when no longer needed for rigidity. In other embodiments, main elongated element 112 is relatively flexible along all or most of its length, by using a flexible polymer or other flexible material to form main elongated element 112. In these embodiments, core wire 128 may run along an entire length of main elongated element 112 and may vary in diameter along the length so as to provide increased rigidity at proximal end 114. In some embodiments, the flexible shaft may also be braided or otherwise strengthened to provide sufficient rigidity.

In embodiments of the present invention, core wire 128 has a portion positioned within main elongated element 112, referred to herein as internal core wire 130, and a portion positioned external to main elongated element 112, referred to herein as external core wire 132. For embodiments wherein main elongated element 112 is comprised of a relatively flexible distal portion and a relatively rigid proximal portion, internal core wire attachment point 144 is located at an interface between the stiff proximal portion and the flexible distal portion, for example, a distal end of the hypotube. In embodiments wherein main elongated element is mostly or completely comprised of flexible material, internal core wire attachment point 144 is located at proximal end 114 of system 100. However, it should be readily apparent that internal core wire attachment point 144 may be located at any location along the length of main elongated element 112. Moreover, multiple internal core wire attachment points 144 are included. At a location proximal to balloon 124, internal core wire 130 exits main elongated element 112 and becomes external core wire 132. This location is referred to herein as a core wire exit point 142. In one embodiment, core wire exit point 142 is at a distal end of main elongated element 112 (but in most cases proximal to balloon 124). In other embodiments, core wire exit point 142 is at other locations along main elongated element 112. Distal to core wire exit point 142, external core wire 132 is positioned alongside balloon 124, and a distal end of external core wire 132 is attached to a distal tip 125 of balloon 124. Several attachment or bonding locations provide transmission of forces through the length of the catheter, and thus enhance overall torquability and rotatability. In particular, bonding can be done at any or all of the following locations: at a distal tip of balloon 124, at core wire exit point 142, and at internal core wire attachment point 144. Additional attachment points may be included as well. It should be noted that the use of an internal core wire makes it possible to have a longer flexible (polymeric or other) portion or even a completely flexible shaft, enhancing overall flexibility of system 100.

System 100 further includes a distal connecting element 138 at distal tip 125 of balloon 124. Distal connecting element 138 is a short rail, extending 2-20 mm, and in some embodiments approximately 10 mm, and may be bonded to distal tip 125 such that the proximal end of distal connecting element 138 is distal to balloon 124. A three-way bond may be used to attach distal connecting element 138, balloon 124 and core wire 132 all together. Distal connecting element 138 may be tapered toward its distal end to facilitate passage through tight stenoses. Distal connecting element 138 is aligned with auxiliary elongated element 118, such that guidewire 48 may be positioned through distal connecting element 38 and further through auxiliary elongated element 118, and out through auxiliary elongated element proximal exit point 121. Thus, only one guidewire is used in the configuration shown in FIG 3. This design provides a single guidewire enclosure split into two sections - one at the distal end and one at the proximal end of balloon 124 - in order to reduce the profile of system 100 in the vicinity of balloon 124 during introduction of system 100 into a vessel. Guidewire 48, while positioned within distal connecting element 138 and auxiliary elongated element 118, can serve as a focused force to help crack difficult lesions and may also be used as a tracking guidewire for advancing system 100 into the vessel.

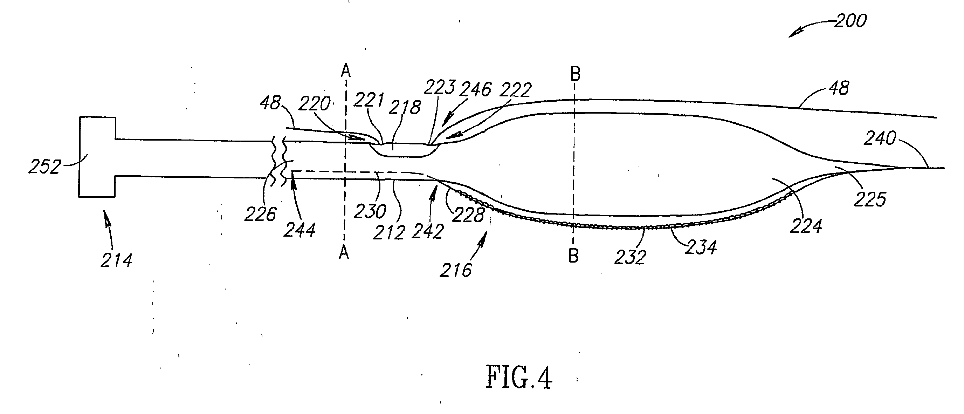

Reference is now made to FIG. 4, which is a schematic illustration of a system 200 for treatment of a vessel, in accordance with yet additional examples, The embodiment shown in FIG. 4 has a reduced profile due to the use of a fixed wire balloon, and may be particularly useful for smaller peripheral vessels such as infra-popliteal vessels, for example. System 200 includes a main elongated element 212 having a proximal end 214 and a distal end 216. In some embodiments of the present invention, main elongated element 212 is a catheter shaft. A balloon 224 is positioned at distal end 216 of main elongated element 212. Balloon 224 can be comprised of a variety of diameters, ranging from 1.25-10.0 mm, for example, and a variety of lengths, ranging from 10 mm to 30 cm, for example. Long balloons may be particularly useful for treating peripheral lesions, which often have long diseased portions. In the embodiment depicted in FIG. 4, balloon 224 is a fixed wire balloon. In one embodiment, balloon 224 is a fixed wire balloon as is commonly known in the art. An example of such a balloon is the type used for the AceTM Balloon Catheter of Boston Scientific Corporation (Natick, MA, USA). In another embodiment, balloon 224 is any balloon with a fixed wire attached thereto. System 200 further includes an auxiliary elongated element 218 configured to receive a guidewire 48 therethrough. Auxiliary elongated element 218 has a proximal end 220 with a proximal exit point 221 for guidewire 48 and a distal end 222 with a distal exit point 223 for guidewire 48. In some embodiments, at least a portion of auxiliary elongated element 218 is positioned within main elongated element 212 so as to reduce the outer profile of system 200. Distal end 222 of auxiliary elongated element 218 is proximal to balloon 224 such that guidewire 48, when positioned through auxiliary elongated element 218, exits distal exit point 223 and runs alongside and external to balloon 224. This configuration provides for a focused force element alongside balloon 224, as will be described further hereinbelow. In some embodiments, such as the one shown in FIG. 4, auxiliary elongated element 218 is relatively short, extending 5-30 cm, and in some embodiments approximately 20 cm. This configuration enables rapid exchange in cases when system 200 may need to be retracted and a different device reinserted over guidewire 48. In other embodiments, auxiliary elongated element 218 may continue proximally along the entire length of main elongated element 212 for an over-the-wire configuration, such as described above with reference to FIG. 1A. In one embodiment, guidewire 48 positioned through distal exit point 223 forms a crotch point 246 at or near a proximal end of balloon 224. The presence of a crotch point may be useful, for example, for anchoring system 200 within a side branch to avoid slippage within the vessel to be treated, or to provide for precise positioning of system 100 at a bifurcation.

In some embodiments, main elongated element 212 is stiffer proximally than distally. This may be accomplished, for example, by using a metal hypotube in the proximal portion and a polymer or other flexible material in the distal portion. This configuration provides more flexibility at the distal end to allow for easier maneuverability through tortuous vessels, while maintaining rigidity at a proximal end for pushability. However, if the distal portion of main elongated element 212 is too flexible, it will be difficult to push through the vessels. Thus, system 200 further includes a core wire 228, which provides enhanced pushability of system 200 without significantly reducing the flexibility of system 200. Core wire 228 is provided in the flexible portion, and may terminate at the stiff portion when no longer needed for rigidity. In other embodiments, main elongated element 212 is relatively flexible along all or most of its length, by using a flexible polymer or other flexible material to form main elongated element 212. In these embodiments, core wire 228 may run along an entire length of main elongated element 212 and may vary in diameter along the length so as to provide increased rigidity at proximal end 214. In some embodiments, the flexible shaft may also be braided or otherwise strengthened to provide sufficient rigidity.

In embodiments of the present invention, core wire 228 has a portion positioned within main elongated element 212, referred to herein as internal core wire 230, and a portion positioned external to main elongated element 212, referred to herein as external core wire 232. For embodiments wherein main elongated element 212 is comprised of a relatively flexible distal portion and a relatively rigid proximal portion, internal core wire attachment point 244 is located at an interface between the stiff proximal portion and the flexible distal portion, for example, a distal end of the hypotube. In embodiments wherein main elongated element is mostly or completely comprised of flexible material, internal core wire attachment point 244 is located at proximal end 214 of system 200. However, it should be readily apparent that internal core wire attachment point may be located at any location along the length of main elongated element 212. Moreover, multiple internal core wire attachment points 244 are included. At a location proximal to balloon 224, internal core wire 230 exits main elongated element 212 and becomes external core wire 232. This location is referred to herein as a core wire exit point 242. In one embodiment, core wire exit point 242 is at a distal end of main elongated element 212 (but in most cases proximal to balloon 224). In other embodiments, core wire exit point 242 is at other locations along main elongated element 212. Distal to core wire exit point 242, external core wire 232 is positioned alongside balloon 224, and a distal end of external core wire 232 is attached to a distal tip 225 of balloon 224. Several attachment or bonding locations provide transmission of forces through the length of the catheter, and thus enhance overall torquability and rotatability. In particular, bonding can be done at any or all of the following locations: at a distal tip of balloon 224, at core wire exit point 242, and at internal core wire attachment point 244. Additional attachment points may be included as well. It should be noted that the use of an internal core wire makes it possible to have a longer flexible (polymeric or other) portion or even a completely flexible shaft, enhancing overall flexibility of system 200. In some embodiments, external core wire 232 and fixed wire 240 are comprised of the same wire. In other embodiments, some or all of external core wire 232 and fixed wire 240 are separate pieces of wire which are connected at the distal tip of balloon 224.

In all of the systems described above, a hydrophilic coating may be added externally to provide ease of insertion.

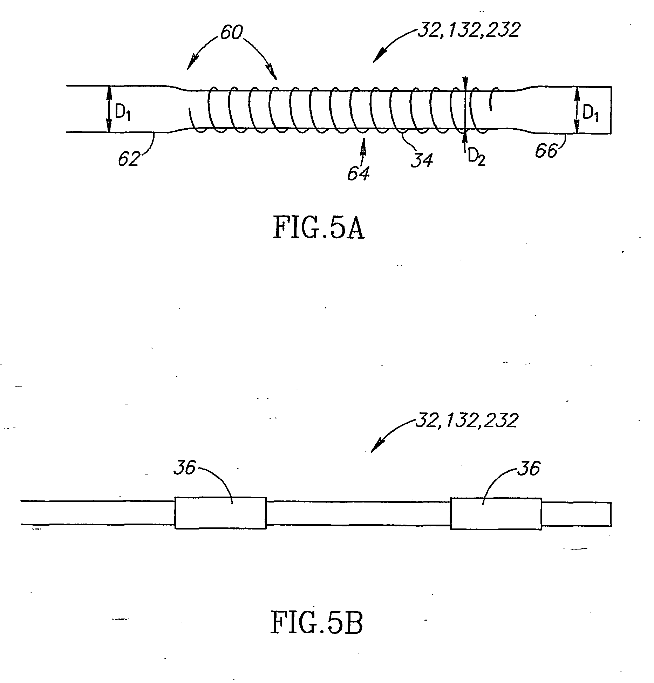

Reference is now made to FIG. 5A and FIG. 5B, which are schematic illustrations of external core wire portion 32, 132, 232 in accordance with embodiments of the present invention. As shown in FIG. 5A, external core wire portion 32, 132 or 232 is configured with a wire portion 60 and a coil 34. Wire portion 60 includes a proximal wire section 62, a mid-wire section 64 and a distal wire section 66. Proximal and distal wire sections 62 and 66 both have a diameter D1 which is greater than a diameter D2 of mid-wire section 64. Coil 34 is wrapped around mid-wire section 64. When in position on system 10, mid-wire section 64 with coil 34 runs alongside balloon 24. This configuration provides enhanced flexibility as well as gripping at the lesion so that slippage of balloon 24, 124, 224 against the lesion is reduced. Moreover, in some embodiments, coil 34 is comprised of radiopaque material, and thus acts as a marker for positioning of system 10, 100 or 200.

Reference is now made to FIG. 5B, which is an illustration of external core wire portion 32 in accordance with another embodiment of the present invention. Core wire 32 is a wire having at least one radiopaque marker 36 thereon. Multiple markers 36 may be used, and may be spaced at optimal locations such as at a proximal end and a distal end of balloon 24, for example.

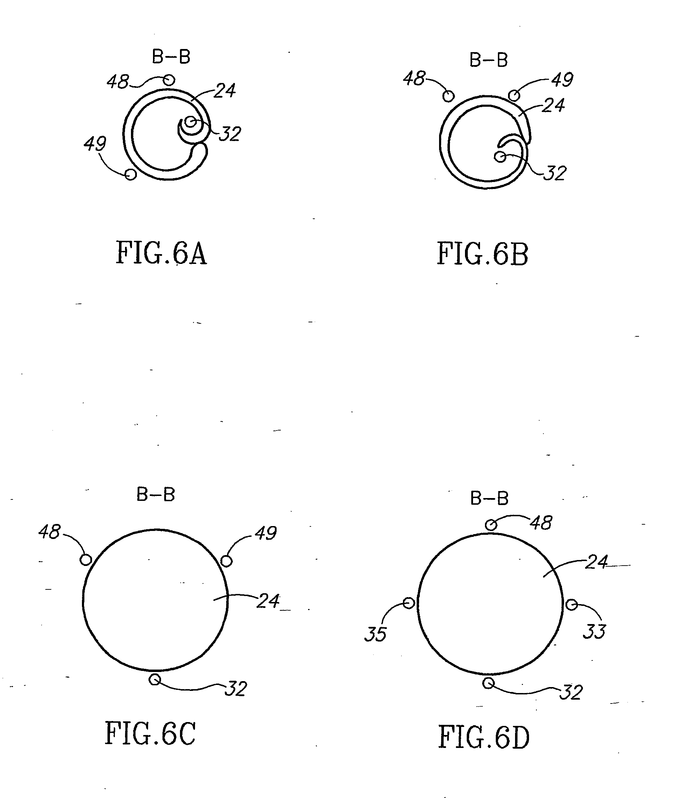

Although external core wire portion 32 is positioned external to balloon 24 when balloon 24 is in its inflated state, as shown in FIGS. 1A, 2, 3 and 4, when balloon 24 is in its deflated state (i.e., during insertion of system 10 into the body), external core wire portion 32 may be positioned within folds of balloon 24. Reference is now made to FIG. 6A-6D, which are cross-sectional illustrations along line B-B of system 10 showing external core wire portion 32, guidewire 48, tracking guidewire 49, and balloon 24 in its deflated state (FIGS. 6A and 6B) and its inflated state (FIGS. 6C and 6D). It should be readily apparent that similar configurations are possible for systems 100 and 200 as well. As shown in FIGS. 6A and 6B, when balloon 24 is in its deflated configuration, external core wire portion 32 is positioned within folds of balloon 24. If a guidewire 48 and/or tracking guidewire 49 are present, guidewire 48 and tracking guidewire 49 can be seen alongside balloon 24. As shown in FIG. 6C, when balloon 24 is expanded, external core wire portion 32 is positioned alongside balloon 24. The external position of external core wire portion 32 with respect to balloon 24 provides an area of focused force for cracking or breaking up hard or difficult lesions. Guidewire 48 and tracking guidewire 49 may be used to provide an additional area of focused force. In some embodiments, guidewire 48 is positioned at a rotational distance from external core wire portion 32 so as to provide multiple areas of focused force around system 10. For example, auxiliary elongated element 18 may be positioned approximately 180 degrees from external core wire portion 32, or approximately 120 degrees from external core wire portion 32 and approximately 120 degrees from tracking guidewire 49, although it should be readily apparent that many different rotational distances are possible.

Reference is now made to FIG. 6D, which is a cross-sectional illustration along line B-B, in accordance with another embodiment. In this embodiment, additional external core wires 33 and 35 are present as well. Although shown with three external core wires, any suitable number of core wires may be used In one embodiment, core wire 28 is split into multiple wires at core wire exit point 42, and the multiple core wires are bundled together at distal end 16 of system 10. In an alternative embodiment, multiple core wire exit points 42 are spaced around main elongated element 12, and multiple core wires exit through the multiple core wire exit points. They are then bundled together at distal tip 25 of balloon 24.

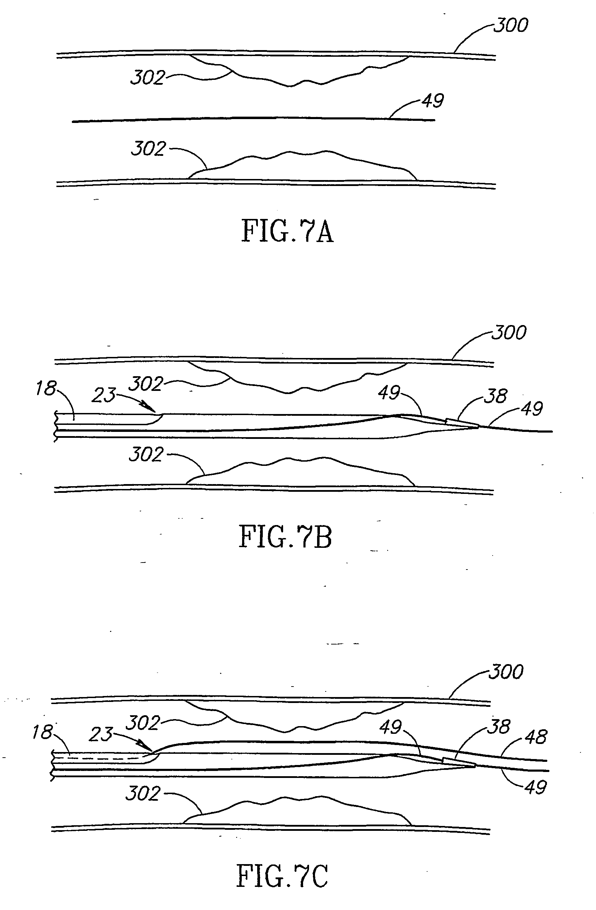

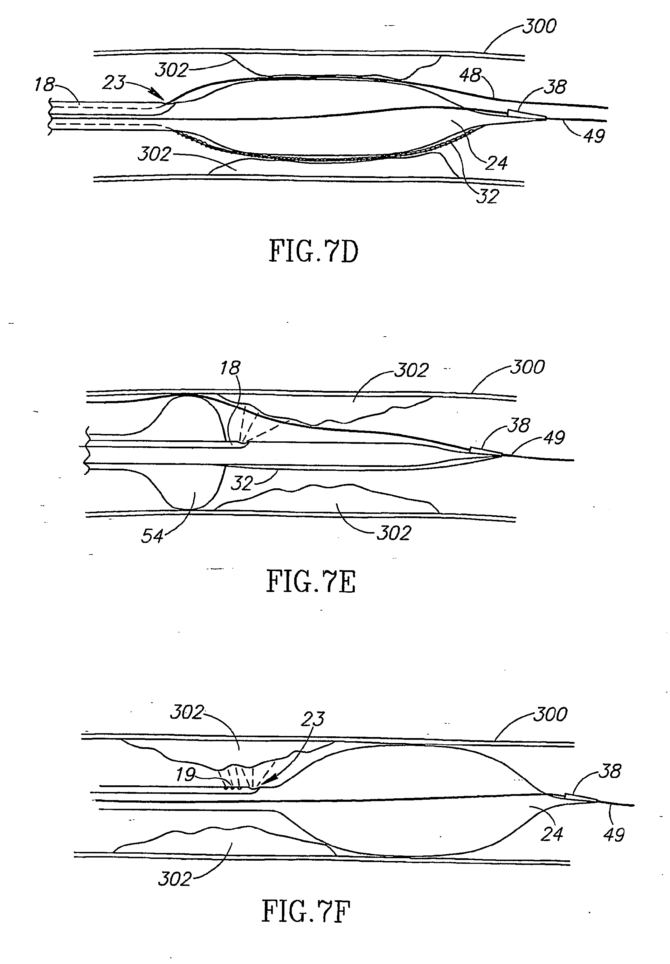

Reference is now made to FIGS. 7A-7E, which are schematic illustrations of the steps of a method of treating a vessel, in accordance with embodiments of the present invention. A vessel 300 having a lesion 302 is accessed via tracking guidewire 49 as shown in FIG. 7A. Tracking guidewire 49 is backloaded onto system 10 by placing tracking guidewire 49 through distal connecting element 38, and system 10 is advanced over tracking guidewire 49 to the vicinity of lesion 302, as shown in FIG. 7B. Next, an additional guidewire 48 may be positioned through auxiliary elongated element 18, and advanced until a distal end of guidewire 48 is distal to balloon 24, as shown in FIG. 7C. In some instances, when guidewire 48 is difficult to advance to this distal location, system 10 may be advanced distally past lesion 302, such that distal exit point 23 of auxiliary elongated element 18 is beyond lesion 302. Guidewire 48 is then advanced through auxiliary elongated element 18. System 10 may then be pulled back proximally so that guidewire 48 and tracking guidewire 49 are adjacent balloon 24 and are in a vicinity of lesion 302. Balloon 24 is then expanded, as shown in FIG. 7D. Expansion of balloon 24 causes external core wire portion 32 to be released from within folds of balloon 24. Expansion of balloon 24 further causes guidewire 48, tracking guidewire 49 and external core wire portion 32 to be pushed up against lesion 302 in three separate rotational positions around the vessel and the lesion. The presence of guidewire 48, tracking guidewire 49, and/or external core wire portion 32 provides a focused force to enable the user to crack hard lesions at low pressure before balloon 24 is fully inflated. Doing so allows vessel stretching to occur at a lower strain rate, thus minimizing the trauma associated with balloon dilatation.

In some embodiments, auxiliary elongated lumen 18 may further be used to provide an item or substance to the vessel. Reference is now made to FIG. 7E, which is a schematic illustration of system 10 positioned inside vessel 300. After the lesion has been cracked or pushed open via balloon 24 and/or external core wire portion 32 and/or guidewire 48, and/or tracking guidewire 49, balloon 24 may then be deflated. In some embodiments, guidewire 48 is retracted to provide an open lumen for delivery of an object or drug to vessel 300. Occlusion balloon 54 is inflated, blocking the portion of vessel 300 which is proximal to occlusion balloon 54. Then, a drug, contrast media or other treatment device may be inserted through auxiliary elongated element 18 and used to treat vessel 300. In some embodiments, after deflating balloon 24, system 10 is advanced past the lesion, occlusion balloon 54 is inflated and treatment is provided to a portion of vessel 300 which is distal to lesion 302. In yet another embodiment, as shown in FIG. 1A, system 10 does not have occlusion balloon 54. After deflating balloon 24, system 10 is advanced past the lesion. Balloon 24 is reinflated at low pressure to occlude vessel 300, and treatment is provided to a portion of vessel 300 that is distal to lesion 302. In some embodiments, ports 19 may provide additional access for treatment of the vessel by spraying treatment solution, for example.

In some embodiments, auxiliary elongated element 18 may be used to introduce a "buddy wire" for tortuous vessels. The "buddy wire" concept is known in the art, and involves introducing a secondary wire alongside a catheter to help straighten out curved vessels and ease the way for the catheter. However, by using a system such as the ones described herein, the "buddy wire" may be introduced within the catheter, minimizing the risk of puncture of the vessel or entanglement of the buddy wire with the catheter. Moreover, systems of the present invention may also be used to introduce a second wire for bifurcations, wherein guidewire 48 introduced through auxiliary elongated element 18 and tracking guidewire 49 may both remain in the vessel. When the system is removed from the body, guidewire 48 is prevented from entanglement with tracking guidewire 49 since guidewire 48 is positioned within auxiliary elongated element 18. Thus, any crossing over which may occur is automatically straightened out during removal of system 10. An additional use of system 10 is in cases where a practitioner encounters a "false lumen". That is, if tracking guidewire 49 encounters an area which is not a true lumen, an additional guidewire 48 may be introduced through system 10 and through the true lumen. System 10 may then be retracted proximally, and advanced over guidewire 48 to cross the lesion.

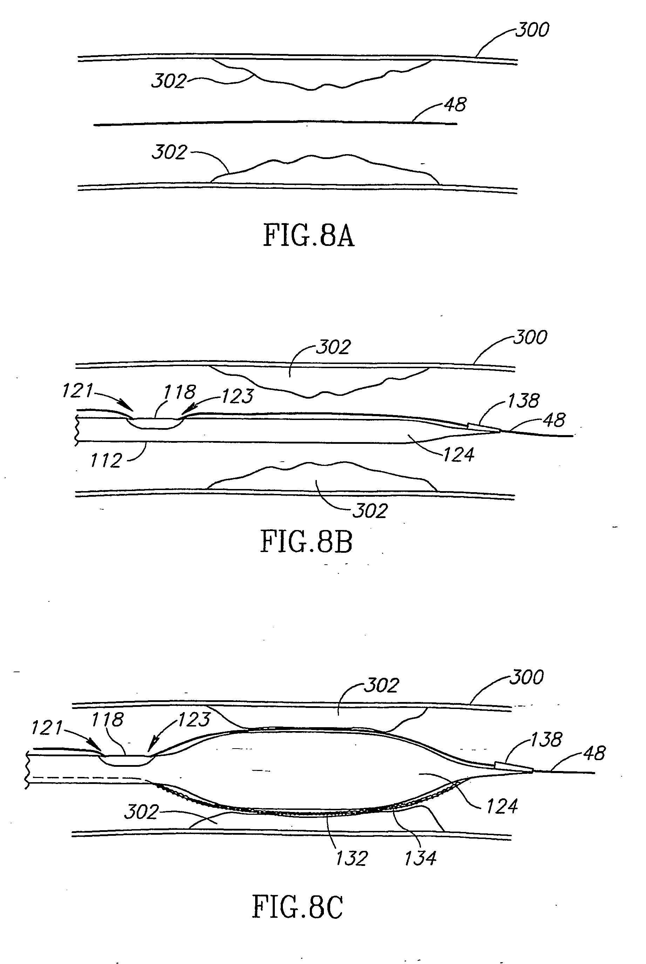

Reference is now made to FIGS. 8A-8C, which are schematic illustrations of the steps of a method of treating a vessel, in accordance with examples. A vessel 300 having a lesion 302 is accessed via guidewire 48, as shown in FIG. 8A. Guidewire 48 is backloaded onto system 100 by placing guidewire 48 through distal connecting element 138, and further positioning guidewire 48 through auxiliary elongated element 118, as shown in FIG. 8B. In some embodiments, an introducer is used to help place guidewire 48 into distal exit point of auxiliary elongated element 118. The introducer may be, for example, a mandrel having a female end, which is pre-loaded into both auxiliary elongated element 118 and distal connecting element 138. When guidewire 48 is backloaded into distal connecting element 138, the proximal end of guidewire 48 is positioned within the female end of the mandrel. The mandrel may then be pulled back proximally, leading guidewire 48 into auxiliary elongated element 118. Guidewire 48 is thus positioned through both distal connecting element 138 and through auxiliary elongated element 118, and exits through auxiliary elongated element proximal exit point 121, which may be relatively close to auxiliary elongated element distal exit point 123 for rapid exchange as shown in FIG. 8B, or may be at proximal end 114 of main elongated element 112 for an over-the-wire configuration. System 100 is advanced over guidewire 48, and positioned such that balloon 124 is adjacent lesion 302, as shown in FIG. 8B. It should be noted that external core wire 132 is not shown in FIG. 8B during insertion, since it is folded into balloon 24. Balloon 124 is then inflated, which pushes both guidewire 48 and external core wire 132 up against lesion 302. The presence of guidewire 48 and/or external core wire 132 provides a focused force to enable the user to crack hard lesions at low pressure before balloon 124 is fully inflated. Doing so allows vessel stretching to occur at a lower strain rate, thus minimizing the trauma associated with balloon dilatation.

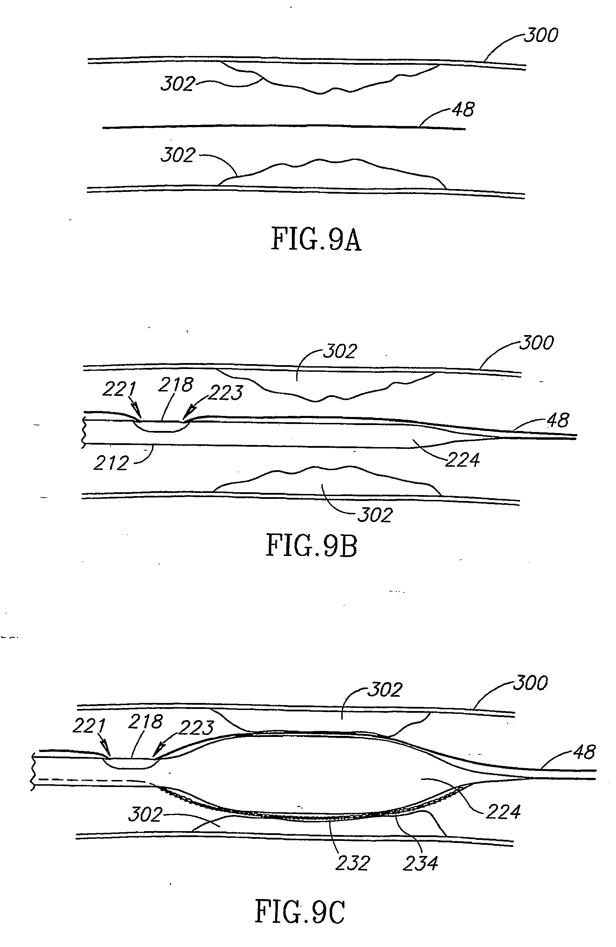

Reference is now made to FIGS. 9A-9C, which are schematic illustrations of the steps of a method of treating a vessel, in accordance with examples. A vessel 300 having a lesion 302 is accessed via guidewire 48. Guidewire 48 is backloaded onto system 200 by placing guidewire 48 through auxiliary elongated element 218. Guidewire 48 exits through auxiliary elongated element proximal exit point 221, which may be relatively close to auxiliary elongated element distal exit point 223 for rapid exchange as shown in FIGS. 9B and 9C, or may be at proximal end 214 of main elongated element 212 for an over-the-wire configuration. System 200 is advanced over guidewire 48, and positioned such that balloon 224 is adjacent lesion 302, as shown in FIG. 9B. It should be noted that external core wire 232 is not shown in FIG. 9B during insertion, since it is folded into balloon 224. Balloon 224 is then inflated, as shown in FIG. 9C, which pushes both guidewire 48 and external core wire 232 up against lesion 302. The presence of guidewire 48 and/or external core wire 232 provides a focused force to enable the user to crack hard lesions at low pressure before balloon 224 is fully inflated. Doing so allows vessel stretching to occur at a lower strain rate, thus minimizing the trauma associated with balloon dilatation. Alternatively, instead of introducing a guidewire, fixed wire 240 is used to cross the lesion. In this embodiment, auxiliary elongated element 218 may optionally not be included. Balloon 224 is then expanded, and external core wire 232 provides the focused force. If auxiliary elongated element 218 is present, a guidewire 48 may additionally be introduced through auxiliary elongated element 218 to provide additional focused force. These forces may be useful in treating a variety of lesions, including those found at renal or peripheral vessels, and may be useful for procedures requiring high forces such as valvuloplasty. It should be readily apparent that when auxiliary elongated element 218 is included, it may also be used as a conduit to provide objects, treatment drugs, contrast media, guidewires, etc. to the vessel.

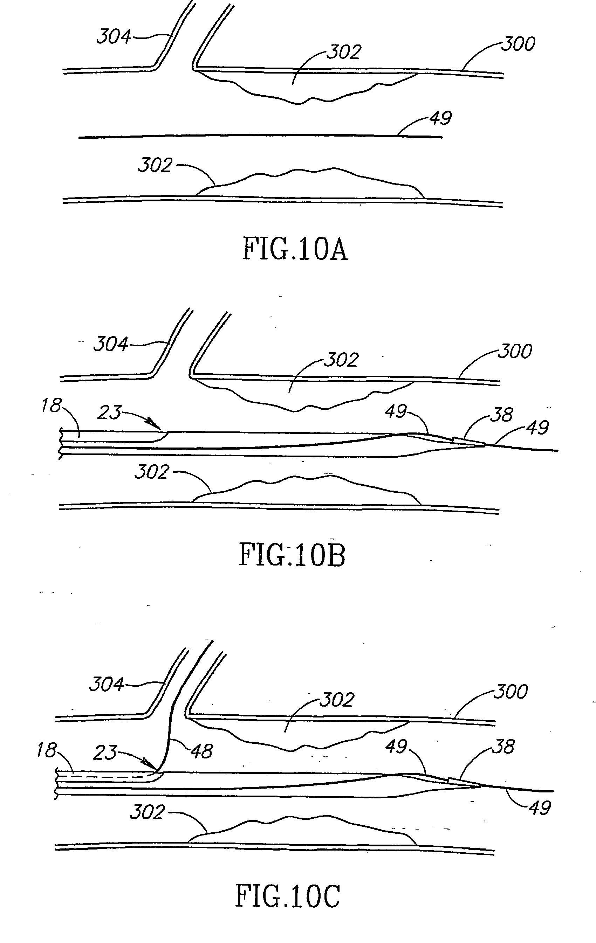

In some embodiments, the systems of the present invention may be used to treat vessels at a bifurcation. Reference is now made to FIGS. 10A-10C, which are schematic illustrations of the steps of a method for treating a bifurcated vessel. First, tracking guidewire 49 is introduced into the main vessel 300, as shown in FIG. 10A. Next, system 10 is advanced over tracking guidewire 49 by backloading tracking guidewire 49 through distal connecting element 38, as shown in FIG. 10B. A guidewire 48 may then be advanced through auxiliary elongated element 18 and into a branch vessel 304. The main vessel lesion 302 may then be treated by inflating balloon 24, while branch vessel 304 is protected in case of plaque shift or additional lesion portions extending into branch vessel 304. In alternative embodiments, system 100 is advanced over a guidewire 48 by backloading guidewire 48 into both distal connecting element 38 and auxiliary elongated element 18. After treatment of lesion 302 in main vessel 300, guidewire 48 may be pulled back proximally and introduced into branch vessel 304. The balloon is deflated, the catheter is retracted along the guidewire, and the system is introduced into the branch vessel. The balloon may then be reinflated so as to compress the lesion in the branch vessel. In an alternative method, the guidewire is introduced into the branch vessel, and the catheter is advanced over the guidewire past the bifurcation and into the main vessel. The main vessel lesion is then treated by inflating the balloon and compressing the lesion. The balloon is deflated, the catheter is retracted, and introduced into the branch vessel such that the guidewire is positioned alongside the balloon. Upon inflation of the balloon, the guidewire is compressed into the lesion site, and provides a focused force to enable the user to crack hard lesions at low pressure before the balloon is fully inflated. This alternative method is possible using system 200 with fixed wire 240, since fixed wire 240 may be used to cross the lesion at the main vessel while guidewire 48 is positioned in the branch vessel.

While certain features of the present invention have been illustrated and described herein, many modifications, substitutions, changes, and equivalents may occur to those of ordinary skill in the art For example, a catheter for uses other than expansion of a balloon and/or delivery of a stent may be used with the device of the present invention, such as a catheter for drug delivery at an ostium, for cauterization, or for any other treatment.

## Claims

1. A device (10) for introduction into a vessel, the device comprising:
a main elongated element (12) having a main elongated element proximal end (14) and a main elongated element distal end (16);
a balloon (24) positioned at said main elongated element distal end (16);
wherein an interior portion of the main elongated element (12) serves as an inflation lumen (26) for providing fluid communication between an inflation port (52) located at the proximal end (14) of the main elongated element (12) and the balloon (24) located at the distal end (16) of the main elongated element (12);
a core wire (28) comprising an internal core wire portion (30) positioned within said main elongated element (12) and an external core wire portion (32) positioned distally with respect to said internal core wire portion, said external core wire portion external to and running alongside said balloon (24); **characterised by** an auxiliary elongated element (18) configured to receive a guidewire therethrough, the auxiliary elongate element having an auxiliary elongated element proximal end (20) and an auxiliary elongated element distal end (22); said auxiliary elongated element distal end (22) being positioned proximal to said balloon (24); and said core wire (28) being attached to said main elongated element at multiple core wire attachment points (44).

2. The device of claim 1, further comprising a distal connecting element (38) positioned at a distal end of said balloon (24), wherein said distal connecting element (38) is at a rotational distance from said auxiliary elongated element (18).

3. The device of claim 2, wherein said distal connecting element (38) and said auxiliary elongated element (18) are at rotational distances from said external core wire portion (32).

4. The device of claim 1, wherein said balloon (24) is a fixed wire balloon.

5. The device of claim 1, wherein said auxiliary elongated element proximal end (121) is positioned at a point along said main elongated element (112), such that a guidewire (48) placed through said auxiliary elongated element (118) may exit at said auxiliary elongated element proximal end for rapid exchange, rather than the proximal exit point being located at a proximal end of the main elongated element as with an over-the-wire configuration.

6. The device of claim 1, wherein said main elongated element (12) is a catheter shaft.

7. The device of claim 2, wherein said rotational distances are between 60 and 120 degrees.

8. The device of claim 1, further comprising a distal connecting element (38) positioned at a distal end of said balloon,wherein said distal connecting element (38) is in line with said auxiliary elongated element (18).

9. The device of claim 1, wherein said auxiliary elongated element (18) is positioned at least partially inside of said main elongated element (12).

10. The device of claim 1, wherein the device is an over-the-wire catheter and wherein said auxiliary elongated element (18) runs along an entire length of the device and, preferably, further comprising an occlusion balloon (54) positioned proximal to said auxiliary elongated element distal end (22).

11. The device of claim 1, wherein said external core wire portion (32) further comprises a coil wrapped around at least a portion thereof, said coil-wrapped portion being positioned adjacent to said balloon (24).

12. The device of claim 1, wherein said external core wire portion (32) comprises at least one radiopaque marker (36).

13. The device of claim 1, wherein said main elongated element (12) comprises a flexible portion (30) and wherein said internal core wire portion is positioned along an entire length of said flexible portion and, preferably, wherein said flexible portion comprises an entire length of said main elongated element.

14. The device of claim 1, wherein said main elongated element has a stiffer proximal portion than a distal portion.

15. The device of claim 14, wherein the proximal portion is provided by a metal hypotube and the distal portion is provided by a polymer.

16. The device of claim 14, or 15, wherein the internal core wire is positioned along an entire length of said proximal portion and preferably wherein the internal core wire terminates at the stiff portion.

## Patentansprüche

1. Vorrichtung (10) zum Einführen in ein Gefäß, die Vorrichtung mit:
einem länglichen Hauptelement (12), das ein proximales längliches Hauptelementende (14) und ein distales längliches Hauptelementende (16) aufweist;
einem Ballon (24), der an dem distalen länglichen Hauptelementende (16) angeordnet ist;
wobei ein innerer Abschnitt des länglichen Hauptelements (12) als ein Befüllungslumen (26) dient, und zwar zum Bereitstellen einer durchgehenden Verbindung zwischen einer Befüllungsöffnung (52), die an dem proximalen Ende (14) des länglichen Hauptelements (12) angeordnet ist, und dem Ballon (24), der an dem distalen Ende (16) des länglichen Hauptelements (12) angeordnet ist;
einem Kerndraht (28) mit einem inneren Kerndrahtabschnitt (30), der in dem länglichen Hauptelement (12) angeordnet ist, und einem äußeren Kerndrahtabschnitt (32), der in Bezug zu dem inneren Kerndrahtabschnitt distal angeordnet ist, wobei der äußere Kerndrahtabschnitt außerhalb und entlang des Ballons (24) verlaufend angeordnet ist;
**gekennzeichnet durch** ein längliches Hilfselement (18), das eingerichtet ist, **durch** sich hindurch einen Führungsdraht zu empfangen, wobei das längliche Hilfselement ein proximales längliches Hilfselementende (20) und ein distales längliches Hilfselementende (22) aufweist und der Kerndraht (28) an mehreren Kerndrahtanbringpunkten (44) an dem länglichen Hauptelement angebracht ist.

2. Vorrichtung nach Anspruch 1, des Weiteren mit einem distalen Verbindungselement (38), das an einem distalen Ende des Ballons (24) angeordnet ist, wobei das distale Verbindungselement (38) einen Rotationsabstand von dem länglichen Hilfselement (18) aufweist.

3. Vorrichtung nach Anspruch 2, bei der das distale Verbindungselement (38) und das längliche Hilfselement (18) Rotationsabstände von dem äußeren Kerndrahtabschnitt (32) aufweisen.

4. Vorrichtung nach Anspruch 1, bei der der Ballon (24) ein befestigter Drahtballon ist.

5. Vorrichtung nach Anspruch 1, bei der das proximale längliche Hilfselementende (121) an einem Punkt entlang des länglichen Hauptelements (112) angeordnet ist, sodass ein durch das längliche Hilfselement (118) angeordneter Führungsdrahts (48) an dem proximalen länglichen Hilfselementende für einen schnellen Austausch austritt, anstatt dass der proximale Austrittspunkt wie bei einer over-the-wire Konfiguration an einem proximalen Ende des länglichen Hauptelements angeordnet ist.

6. Vorrichtung nach Anspruch 1, bei der das längliche Hauptelement (12) ein Katheterschaft ist.

7. Vorrichtung nach Anspruch 2, bei der die Rotationsabstände zwischen 60 und 120° sind.

8. Vorrichtung nach Anspruch 1, des Weiteren mit einem distalen Verbindungselement (38), das an einem distalen Ende des Ballons angeordnet ist, wobei das distale Verbindungselement (38) mit dem länglichen Hilfselement (18) fluchtet.

9. Vorrichtung nach Anspruch 1, bei der das längliche Hilfselement (18) zumindest teilweise innerhalb des länglichen Hauptelements (12) angeordnet ist.

10. Vorrichtung nach Anspruch 1, bei der die Vorrichtung ein over-the-wire Katheter ist und wobei das längliche Hilfselement (18) entlang einer gesamten Länge der Vorrichtung verläuft und des Weiteren bevorzugt einen Verschlussballon (54) aufweist, der proximal zu dem distalen länglichen Hilfselementende (22) angeordnet ist.

11. Vorrichtung nach Anspruch 1, bei der der äußere Kerndrahtabschnitt (32) des Weiteren eine Spule aufweist, die zumindest um einen Teil davon gewickelt ist, wobei der spulenumwickelte Abschnitt angrenzend an dem Ballon (24) angeordnet ist.

12. Vorrichtung nach Anspruch 1, bei der der äußere Kerndrahtabschnitt (32) zumindest einen röntgenundurchlässigen Marker (36) aufweist.

13. Vorrichtung nach Anspruch 1, bei der das längliche Hauptelement (12) einen flexiblen Abschnitt (30) aufweist, der innere Kerndrahtabschnitt entlang einer gesamten Länge des flexiblen Abschnitts angeordnet ist und wobei der flexible Abschnitt bevorzugt eine gesamte Länge des länglichen Hauptelements aufweist.

14. Vorrichtung nach Anspruch 1, bei der das längliche Hauptelement einen proximalen Abschnitt aufweist, der steifer als ein distaler Abschnitt ist.

15. Vorrichtung nach Anspruch 14, bei der der proximale Abschnitt durch eine Metall-Hyporöhre bereitgestellt ist und der distale Abschnitt durch ein Polymer bereitgestellt ist.

16. Vorrichtung nach Anspruch 14 oder 15, bei der der innere Kerndraht entlang einer gesamten Länge des proximalen Abschnitts angeordnet ist und der innere Kerndraht bevorzugt bei dem steifen Abschnitt endet.

## Revendications

1. Dispositif (10) destiné à être introduit dans un vaisseau, le dispositif comprenant :
un élément allongé principal (12) ayant une extrémité proximale (14) de l'élément allongé principal et une extrémité distale (16) de l'élément allongé principal ;
un ballon (24) positionné au niveau de ladite extrémité distale (16) de l'élément allongé principal ;
dans lequel une partie intérieure de l'élément allongé principal (12) sert de lumière de gonflage (26) pour fournir une communication fluidique entre un orifice de gonflage (52) situé au niveau de l'extrémité proximale (14) de l'élément allongé principal (12) et le ballon (24) situé au niveau de l'extrémité distale (16) de l'élément allongé principal (12) ;
une tige d'armature (28) comprenant une partie (30) de tige d'armature interne positionnée à l'intérieur dudit élément allongé principal (12) et une partie (32) de tige d'armature externe positionnée de manière distale par rapport à ladite partie de tige d'armature interne, ladite partie de tige d'armature externe étant à l'extérieur dudit ballon (24) et longeant ce dernier ;
**caractérisé par** un élément allongé auxiliaire (18) configuré pour recevoir un fil guide à travers celui-ci, l'élément allongé auxiliaire ayant une extrémité proximale (20) de l'élément allongé auxiliaire et une extrémité distale (22) de l'élément allongé auxiliaire ;
ladite extrémité distale (22) de l'élément allongé auxiliaire étant positionnée à proximité dudit ballon (24) ; et ladite tige d'armature (28) étant fixée audit élément allongé principal à différents points de fixation (44) de la tige d'armature.

2. Dispositif de la revendication 1, comprenant en outre un élément de liaison distal (38) positionné au niveau d'une extrémité distale dudit ballon (24), où ledit élément de liaison distal (38) est situé à une distance de rotation dudit élément allongé auxiliaire (18).

3. Dispositif de la revendication 2, dans lequel ledit élément de liaison distal (38) et ledit élément allongé auxiliaire (18) sont situés à des distances de rotation de ladite partie (32) de tige d'armature externe.

4. Dispositif de la revendication 1, dans lequel ledit ballon (24) est un ballon à fil fixe.

5. Dispositif de la revendication 1, dans lequel ladite extrémité proximale (121) de l'élément allongé auxiliaire est positionnée au niveau d'un point le long dudit élément allongé principal (112), de sorte qu'un fil guide (48) placé à travers ledit élément allongé auxiliaire (118) puisse sortir au niveau de ladite extrémité proximale de l'élément allongé auxiliaire pour échange rapide, plutôt qu'au niveau du point de sortie proximal qui est situé au niveau d'une extrémité proximale de l'élément allongé principal comme pour une configuration sur fil.

6. Dispositif de la revendication 1, dans lequel ledit élément allongé principal (12) est une tige de cathéter.

7. Dispositif de la revendication 2, dans lequel lesdites distances de rotation sont comprises entre 60 et 120 degrés.

8. Dispositif de la revendication 1, comprenant en outre un élément de liaison distal (38) positionné au niveau d'une extrémité distale dudit ballon, dans lequel ledit élément de liaison distal (38) est aligné avec ledit élément allongé auxiliaire (18).

9. Dispositif de la revendication 1, dans lequel ledit élément allongé auxiliaire (18) est positionné au moins en partie à l'intérieur dudit élément allongé principal (12).

10. Dispositif de la revendication 1, dans lequel le dispositif est un cathéter sur fil et dans lequel ledit élément allongé auxiliaire (18) s'étend le long du dispositif et, de préférence, comprenant en outre un ballon d'occlusion (54) positionné à proximité de ladite extrémité distale (22) de l'élément allongé auxiliaire.

11. Dispositif de la revendication 1, dans lequel ladite partie (32) de tige d'armature externe comprend en outre une bobine enroulée autour d'au moins une partie de celle-ci, ladite partie à bobine enroulée étant positionnée adjacente audit ballon (24).

12. Dispositif de la revendication 1, dans lequel ladite partie (32) de tige d'armature externe comprend au moins un marqueur radio-opaque (36).

13. Dispositif de la revendication 1, dans lequel ledit élément allongé principal (12) comprend une partie flexible (30) et où ladite partie de tige d'armature interne est positionnée le long d'une longueur entière de ladite partie flexible et, de préférence, où ladite partie flexible comprend une longueur entière dudit élément allongé principal.

14. Dispositif de la revendication 1, dans lequel ledit élément principal allongé présente une partie proximale plus rigide qu'une partie distale.

15. Dispositif de la revendication 14, dans lequel la partie proximale est pourvue d'un hypotube métallique et la partie distale est pourvue d'un polymère.

16. Dispositif de la revendication 14, ou 15, dans lequel la tige d'armature interne est positionnée le long d'une longueur entière de ladite partie proximale et de préférence où la tige d'armature interne se termine au niveau de la partie rigide.
